(19) Europäisches Patentamt
European Patent Office
Office européen des brevets

(11) Veröffentlichungsnummer: **0 184 752**
**A2**

# (12) EUROPÄISCHE PATENTANMELDUNG

(21) Anmeldenummer: **85115278.5**

(22) Anmeldetag: **02.12.85**

(51) Int. Cl.4: **C 07 D 239/48**, A 01 N 43/54

(30) Priorität: **12.12.84 DE 3445293**

(43) Veröffentlichungstag der Anmeldung: **18.06.86**
**Patentblatt 86/25**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Schwamborn, Michael, Dr., von-Lohe-Strasse 9, D-5000 Köln 80 (DE)**
Erfinder: **Kühle, Engelbert, Dr., von-Bodelschwingh-Strasse 42, D-5060 Bergisch-Gladbach 2 (DE)**
Erfinder: **Eue, Ludwig, Dr., Paul-Klee-Strasse 36, D-5090 Leverkusen 1 (DE)**
Erfinder: **Schmidt, Robert R., Dr., Im Waldwinkel 110, D-5060 Bergisch-Gladbach 2 (DE)**
Erfinder: **Santel, Hans-Joachim, Dr., Grünstrasse 9a, D-5090 Leverkusen 1 (DE)**

## (54) Neue 2,4-Diaminopyrimidine.

(57) Die Erfindung betrifft neue 2,4-Diaminopyrimidine der allgemeinen Formel (I)

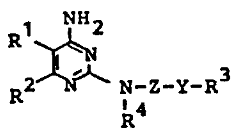

in welcher
$R^1$ für Wasserstoff oder Halogen steht,
$R^2$ für gegebenenfalls durch Halogen substituiertes Alkyl, Halogen oder Alkoxy steht,
$R^3$ für Alkyl steht,
$R^4$ für Wasserstoff oder Alkyl steht,
Y für Sauerstoff oder Schwefel steht und
Z für verzweigtes oder unverzweigtes Alkylen steht,
Verfahren zu ihrer Herstellung und ihre Verwendung als Pflanzenschutzmittel, insbesondere als Herbizide.

0184752



BAYER AKTIENGESELLSCHAFT 5090 Leverkusen, Bayerwerk
Konzernverwaltung RP
Patentabteilung Bi/KM/Kü-c
Ib

Neue 2,4-Diaminopyrimidine

Die vorliegende Erfindung betrifft neue 2,4-Diaminopyrimidine, Verfahren zu ihrer Herstellung sowie ihre Verwendung als Pflanzenschutzmittel, insbesondere als Herbizide.

Es ist bereits bekannt, daß bestimmte 2,4-Diaminopyrimidine, z.B. 2,4-Biscyclopropylamino-5,6-dichlorpyrimidin, als Herbizide eingesetzt werden können (vgl. DE-OS 2 006 145, DE-OS 2 630 140). Ihre Wirkung bei niedrigen Aufwandmengen ist jedoch bei verschiedenen Schadpflanzen nicht befriedigend.

Es wurden nun neue 2,4-Diaminopyrimidine der allgemeinen Formel (I)

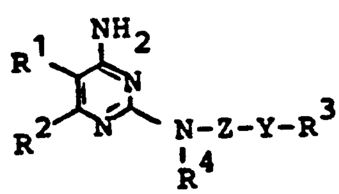

in welcher

$R^1$ für Wasserstoff oder Halogen steht,

$R^2$ für gegebenenfalls durch Halogen substituiertes Alkyl, für Halogen oder Alkoxy steht,

$R^3$ für Alkyl steht,

$R^4$ für Wasserstoff oder Alkyl steht,

Y für Sauerstoff oder Schwefel steht und

Z für verzweigtes oder unverzweigtes Alkylen steht,

aufgefunden.

Weiterhin wurde gefunden, daß man die Pyrimidinderivate der allgemeinen Formel (I) erhält, wenn man entweder

(A) Halogenpyrimidine der allgemeinen Formel (II)

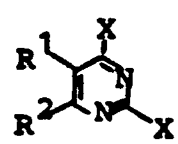

(II)

in welcher

$R^1$ und $R^2$ die oben angegebene Bedeutung haben und X für Halogen (bevorzugt Fluor oder Chlor) steht,

zunächst mit einem Amin der Formel (III)

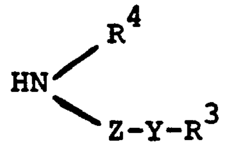

(III)

worin

$R^3$, $R^4$, Y und Z die oben angegebene Bedeutung haben,

in Gegenwart eines säurebindenden Mittels und gegebenenfalls in Gegenwart eines Verdünnungsmittels zu einem Gemisch der isomeren Pyrimidinderivate der allgemeinen Formel (IVa) und (IVb)

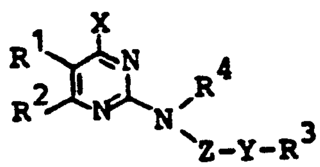

(IVa)

+

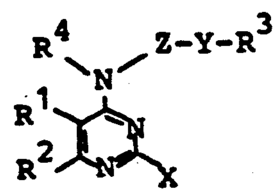

(IVb)

worin

$R^1$, $R^2$, $R^3$, $R^4$, X, Y und Z die oben angegebene Bedeutung haben,

umsetzt (1. Stufe) und anschließend nach Trennung der beiden Isomeren die Pyrimidinderivate der Formel (IVa) in einem weiteren Reaktionsschritt mit Ammoniak (V)

$NH_3$ (V)

in Gegenwart eines säurebindenden Mittels und gegebenenfalls in Gegenwart eines Verdünnungsmittels zu Pyrimidinderivaten der Formel (I) umsetzt (2. Stufe),

oder

(B) 4-Aminopyrimidinderivate der allgemeinen Formel (VI)

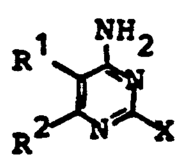

(VI)

worin

$R^1$, $R^2$ und X die oben angegebene Bedeutung haben,

mit einem Amin der Formel (III) - wiederum unter den gleichen Reaktionsbedindungen wie bei Verfahren (A) - zu Pyrimidinderivaten der Formel (I)

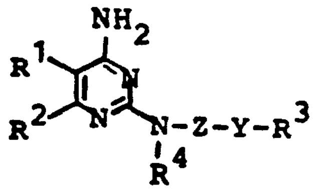 (I)

worin

$R^1$, $R^2$, $R^3$, $R^4$, Y und Z die oben angegebene Bedeutung haben,

umsetzt.

Hierbei erhält man, wenn $R^2$ für Halogen steht, ein Gemisch der isomeren Pyrimidinderivate der allgemeinen Formel (I) und (Ia)

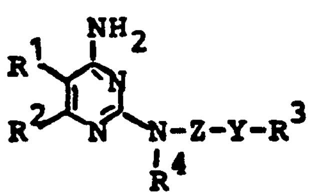

(I)

und

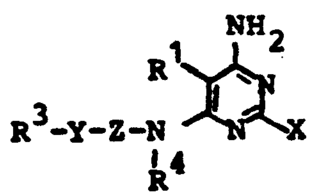

(Ia)

worin

$R^1$, $R^2$, $R^3$, $R^4$, X, Y und Z die oben angegebene Bedeutung haben, aus dem das gewünschte Pyrimidinderivat der allgemeinen Formel (I) abgetrennt wird.

Außerdem wurde gefunden, daß die neuen Pyrimidinderivate der allgemeinen Formel (I) starke herbizide Eigenschaften aufweisen.

Die erfindungsgemäßen neuen Pyrimidinderivate der Formel (I) zeichnen sich gegenüber den vorbekannten Pyrimidinen strukturell insbesondere dadurch aus, daß die 2-Aminogruppe eine geradkettige oder verzweigte, durch Sauerstoff oder Schwefel unterbrochene Alkylkette enthält und daß in 4-Stellung eine unsubstituierte Aminogruppe steht.

Überraschenderweise sind die erfindungsgemäßen Wirkstoffe der Formel (I), bei gleicher Kulturverträglichkeit, deutlich wirksamer als die vorbekannten Pyrimidinderivate, wie z.B. 2,4-Biscyclopropylamino-5,6-dichlorpyrimidin (bekannt aus DE-OS 2 630 140).

Von den erfindungsgemäßen Pyrimidinderivaten der Formel (I) sind bevorzugt diejenigen, in denen

$R^1$ für Wasserstoff, Chlor oder Fluor steht,

$R^2$ für Chlor, Fluor, Alkoxy mit 1-6 C-Atomen oder für gegebenenfalls durch Chlor und/oder Fluor substituiertes Alkyl mit 1-6 C-Atomen steht,

$R^3$ für Alkyl mit 1-6 C-Atomen steht,

$R^4$ für Wasserstoff oder Alkyl mit 1-6 C-Atomen steht,

Y für Sauerstoff oder Schwefel steht und

Z für eine verzweigte oder unverzweigte Alkylengruppe mit 2 bis 10 C-Atomen steht.

Aus dieser Stoffgruppe sind solche Verbindungen der Formel (I) besonders bevorzugt, in denen

$R^1$ für Wasserstoff, Chlor oder Fluor steht,

$R^2$ für Chlor, Fluor, Alkoxy mit 1-3 C-Atomen oder für gegebenenfalls durch Chlor und/oder Fluor substituiertes Alkyl mit 1 oder 2 C-Atomen steht,

$R^3$ für Alkyl mit 1-4 C-Atomen steht,

$R^4$ für Wasserstoff oder Alkyl mit 1-4 C-Atomen steht,

Y für Sauerstoff oder Schwefel steht und

Z für eine verzweigte oder unverzweigte Alkylengruppe mit 2-7 C-Atomen steht.

Verwendet man gemäß Verfahren (A) beispielsweise 2,4,6-Trichlorpyrimidin und 3-Methoxypropylamin als Ausgangsstoffe und setzt das hierbei gebildete 4,6-Dichlor-2-(3-methoxypropylamino)-pyrimidin weiter mit Ammoniak um, so kann der Reaktionsablauf zusammenfassend durch das folgende Formelschema wiedergegeben werden:

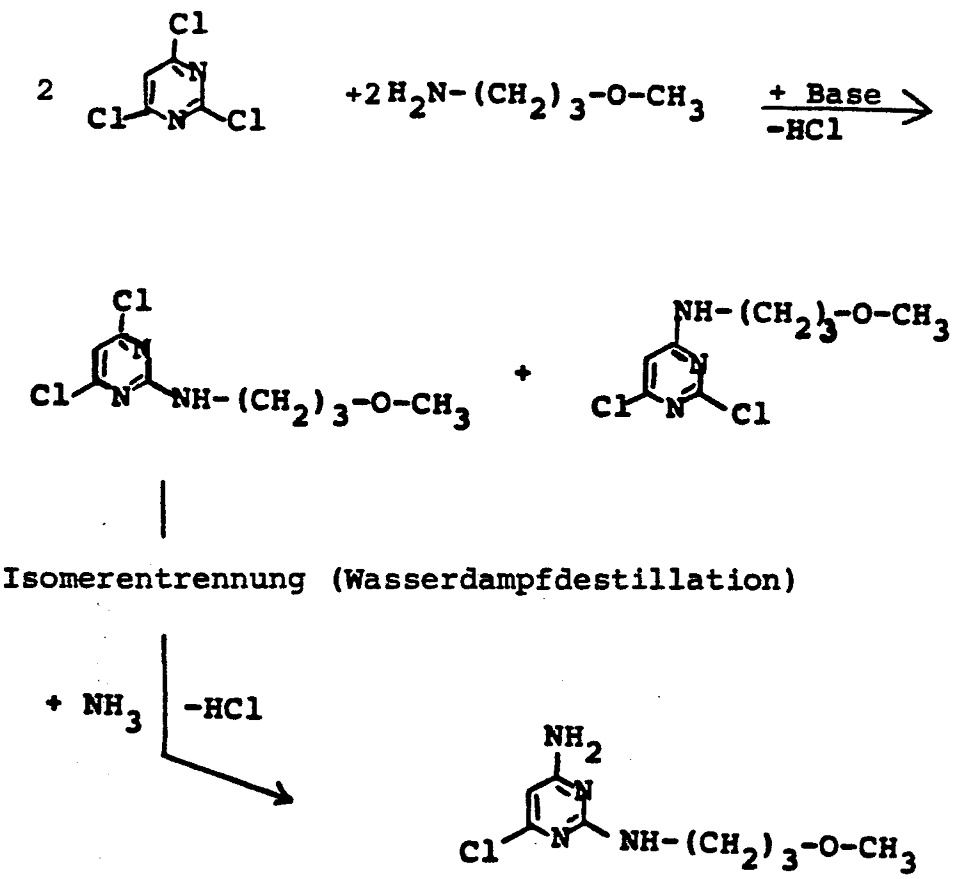


Verwendet man gemäß Verfahren (B) beispielsweise 4-Amino-2,5,6-trichlorpyrimidin als Ausgangsstoff und setzt es mit 3-Methoxypropylamin um, so kann der Reaktionsablauf zusammenfassend durch das folgende Formelschema wiedergegeben werden:

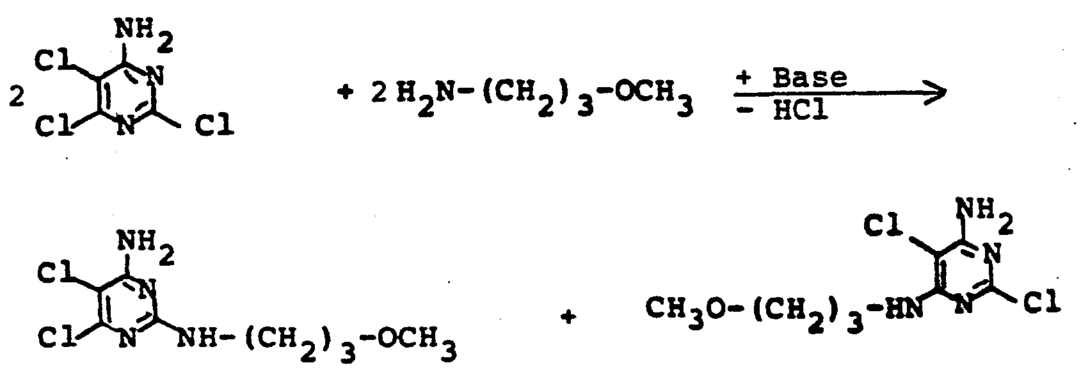


Isolierung durch Isomerentrennung (Kristallisation).

Die als Ausgangsstoffe verwendeten Halogenpyrimidine sind durch die Formel (II) allgemein definiert. In dieser Formel stehen $R^1$ und $R^2$ vorzugsweise bzw. besonders bevorzugt für diejenigen Reste, die schon bei der Beschreibung der erfindungsgemäßen Stoffe der Formel (I) als bevorzugt bzw. besonders bevorzugt für diese Substituenten genannt wurden und X für Chlor oder Fluor. Die Halogenpyrimidine der Formel (II) sind zum Teil bekannt bzw. können nach bekannten Verfahren hergestellt werden (vgl. Beispiele).

Die weiterhin als Ausgangsstoffe verwendeten Amine sind durch die Formel (III) allgemein definiert. In dieser Formel stehen $R^3$, $R^4$, Y und Z vorzugsweise bzw. besonders bevorzugt für diejenigen Reste, die schon bei der Beschreibung der erfindungsgemäßen Stoffe der Formel (I) als bevorzugt bzw. besonders bevorzugt für diese Substituenten genannt wurden. Die Amine der Formel (III) sind bekannt oder lassen sich nach bekannten Verfahren in analoger Weise wie die bekannten Verbindungen herstellen (vgl. z.B. Houben-Weyl, Methoden der organischen Chemie, Band XI/1, S. 548, S. 561 ff., 4. Auflage 1957; US 2 764 615).

Die als Ausgangsstoffe verwendeten 4-Aminopyrimidinderivate sind durch die Formel (VI) allgemein definiert. In dieser Formel stehen $R^1$ und $R^2$ vorzugsweise bzw. besonders bevorzugt für diejenigen Reste, die schon bei der Beschreibung der erfindungsgemäßen Stoffe der Formel (I) als bevorzugt oder besonders

bevorzugt genannt wurden und X für Chlor oder Fluor. Die 4-Aminopyrimidinderivate der Formel (VI) sind zum Teil bekannt bzw. können nach bekannten Verfahren hergestellt werden (vgl. Beispiele).

Als Verdünnungsmittel für die erfindungsgemäßen Verfahrensvarianten (A) und (B) kommen organische Lösungsmittel sowie Wasser infrage. Bevorzugte organische Lösungsmittel sind Kohlenwasserstoffe wie Toluol, aliphatische Ketone wie Aceton, Methylethylketon und Diethylketon, cycloaliphatische Ether wie Tetrahydrofuran oder Dioxan. Auch Gemische verschiedener organischer Lösungsmittel und Gemische von mit Wasser mischbaren organischen Lösungsmitteln mit Wasser sind als Verdünnungsmittel geeignet.

Die erfindungsgemäßen Verfahrensvarianten (A) und (B) können unter Verwendung von Säurebindemitteln durchgeführt werden. Als solche sind Erdalkali- und Alkalimetallhydroxide, wie Calcium-, Natrium- oder Kaliumhydroxid, ferner Ammoniak sowie tertiäre aliphatische Amine wie z.B. Triethylamin, aber auch im Überschuß eingesetztes Amin-Ausgangsprodukt (III) besonders geeignet.

Die Reaktionstemperaturen können bei den erfindungsgemäßen Verfahren in einem größeren Bereich variiert werden. Die erste Verfahrensstufe bei Verfahrensweise (A) wird im allgemeinen bei Temperaturen von -30 bis +150°C, vorzugsweise von -20 bis +50°C durchgeführt; bei der zweiten Verfahrensstufe arbeitet man im allgemeinen bei 80 bis 150°C, bevorzugt bei 90 bis 130°C. Bei Verfahrensweise (B) arbeitet man im allgemeinen bei 80 bis 150°C, bevorzugt bei 90 bis 130°C.

Die Umsetzung wird im Druckbereich von 1 bis etwa 10 bar durchgeführt.

Bei der Durchführung des erfindungsgemäßen Verfahrens (A) setzt man auf 1 Mol Halogenpyrimidin der Formel (II) in der ersten Stufe im allgemeinen 1 bis 1,1 Mol Amin der Formel (III) und 1 bis 1,2 Mol Säurebinder ein, wobei als Säurebinder das Amin (III) verwendet werden kann. Bevorzugt wird unter Einsatz stöchiometrischer Molverhältnisse gearbeitet. Für die zweite Verfahrensstufe sowie Verfahrensvariante (B) gilt Entsprechendes.

Die bei beiden Verfahrensvarianten anfallenden Isomerengemische können in einfacher Weise nach bekannten Methoden getrennt werden, insbesondere durch Umkristallisation, Chromatographie oder Wasserdampfdestillation (vgl. z.B. DE-OS 2 006 145, DE-OS 2 630 140, EP 0 114 575), so daß sich die jeweils gewünschten Isomeren in ausreichend reiner Form isolieren lassen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können - insbesondere im Nachauflaufverfahren - zur selektiven Unkrautbekämpfung in monokotylen Kulturen, z.B. in Mais, Getreide und Reis, und dikotylen Kulturen, z.B. in Baumwolle und Soja, eingesetzt werden. Die neuen Wirkstoffe sind bei gleicher Verträglichkeit sowohl für Mais, Getreide und Reis als auch für Baumwolle und Soja deutlich wirksamer als die vorbekannte Verbindung 2,4-Biscyclopropylamino-5,6-dichlorpyrimidin.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Als Mischpartner bei der Herbizid-Anwendung kommen infrage: Harnstoffe (z.B. Methabenzthiazuron); Diphenylether, Acetanilide (z.B. Alachlor, Metolachlor); Phenoxyalkancarbonsäuren (z.B. 2,4-D, 2,4-DP, MCPA, MCPP und deren Derivate); Aryloxy- und Hetaryloxyphenoxy-propionsäure (z.B. 2-[4-(3,5-Dichlor-2-pyridyloxy)-phenoxy]-propionsäure-(trimethylsilylmethyl)-ester; 2-[4-(3,5-Dichlor-2-pyridyloxy)-phenoxy]-propionsäure-(2,2-diethoxy)-ethylester; 2-[4-(3,5-Dichlor-2-pyridyloxy)-phenoxy]-propionsäure-(2-benzyloxy)-ethylester); Triazine (z.B. Atrazin, Simazin); Triazinone (z.B. Metribuzin und 4-Amino-6-tert.-butyl-3-ethylthio-1,2,4-triazin-5(4H)-on); Triazindione (z.B. Ametridione); Cyclohexandione (z.B. Sethoxydim); Benzonitrile (z.B. Joxynil) und Bentazon.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

0184752

Herstellungsbeispiele

Beispiel 1

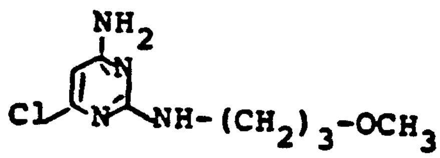

(1)

4-Amino-6-chlor-2-(3-methoxypropylamino)-pyrimidin/nach Verfahren (A):

a) 4,6-Dichlor-2-(3-methoxypropylamino)-pyrimidin / 1. Stufe

Zu 50 g (0,27 Mol) 2,4,6-Trichlorpyrimidin, gelöst in 300 ml Tetrahydrofuran, werden bei -30°C 48 g (0,54 Mol) 3-Methoxypropylamin getropft. Nach Auftauen auf Raumtemperatur und sechsstündigem Nachrühren gibt man das Reaktionsgemisch in Eiswasser, extrahiert mit Dichlormethan, trocknet über Natriumsulfat und entfernt das Solvens. Der resultierende Rückstand wird einer Wasserdampfdestillation unterzogen, wobei 10,6 g (16,6 % der Theorie) des gewünschten Pyrimidins als Feststoff übergehen, Fp. 48°C.

b) 4-Amino-6-chlor-2-(3-methoxypropylamino)-pyrimidin / 2. Stufe

10 g (0,042 Mol) 4,6-Dichlor-2-(3-methoxypropylamino)-pyrimidin werden in 100 ml Dioxan gelöst und mit 60 ml wäßriger Ammoniaklösung versetzt. Nach vierstündigem Erhitzen zum Sieden wird das Reaktionsgemisch mit 1 Liter Eiswasser versetzt und mit Dichlormethan extrahiert. Nach Trocknen über Natriumsulfat und Einengen wird der verbleibende Rückstand mit Waschbenzin aufgekocht, abfiltriert und getrocknet. Man erhält 5,6 g (61,6 % der Theorie) des gewünschten Pyrimidins, Fp. 141°C.

Beispiel 2

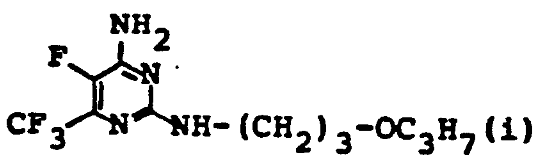

(2)

4-Amino-2-(3-isopropoxypropylamino)-5-fluor-6-trifluormethylpyrimidin / nach Verfahren (B):

5 g (0,025 Mol) 4-Amino-2,5-difluor-6-trifluormethylpyrimidin werden in 150 ml Dioxan gelöst. Hierzu tropft man 2,9 g (0,025 Mol) 3-Isopropoxypropylamin und 2,5 g (0,025 Mol) Triethylamin und erhitzt fünf

Stunden zum Sieden. Das Reaktionsgemisch wird abgekühlt, in Eiswasser gerührt und der resultierende Feststoff wird abgesaugt, getrocknet und aus Waschbenzin umkrisallisiert. Man erhält 4 g (54 % der Theorie) des gewünschten Pyrimidins, Fp. 87°C.

Beispiel 3

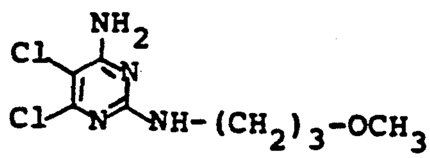

(3)

4-Amino-2-(3-methoxypropylamino)-5,6-dichlorpyrimidin / nach Verfahren (B):

1850 g (9,31 Mol) 4-Amino-2,5,6-trichlorpyrimidin werden in 4 Liter Tetrahydrofuran gelöst. Hierzu tropft man 1659 g (18,62 Mol) 3-Methoxypropylamin über einen Zeitraum von 6 Stunden. Nach fünfstündigem Erhitzen zum Sieden wird die abgekühlte Reaktionsmischung in 20 Liter Wasser eingerührt. Der resultierende Feststoff wird abgesaugt, getrocknet, mit 7 Liter Dichlormethan verrührt und wiederum abgesaugt. Nach zweimaligem Umkristallisieren des so gewonnenen Feststoffs aus Toluol erhält man 743 g (31,8 % der Theorie) des gewünschten Pyrimidins, Fp. 114°C.

Beispiel 4

4-Amino-2-(3-methoxy-1-methylpropylamino)-5,6-dichlor-pyrimidin / nach Verfahren (B):

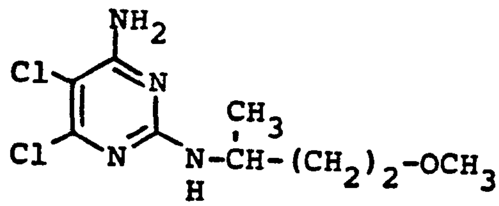

(4)

Zu einer Lösung von 6,5 g (0,033 Mol) 4-Amino-2,5,6-trichlorpyrimidin in 200 ml Toluol tropft man sukzessive 3,4 g (0,033 Mol) 3-Methoxy-1-methylpropylamin und 3,3 g (0,033 Mol) Triethylamin und erhitzt fünf Stunden zum Sieden. Nach dem Abkühlen wird das Reaktionsgemisch mit Wasser gewaschen, die organische Phase über Natriumsulfat getrocknet und eingeengt. Das so erhaltene Rohprodukt wird mittels Chromatographie an Kieselgel (Länge der Säule: 80 cm, Durchmesser: 5 cm) mit Essigester und Petrolether (1:1) gereinigt. Man erhält neben 4-Amino-6-(3-methoxy-1-methylpropylamino)-2,5-dichlorpyrimidin (1. Fraktion, Fp. 62°C) 1,5 g (17,2 % der Theorie) (2. Fraktion, Fp. 96°C) des gewünschten Pyrimidins.

Auf analogem Wege können die in der nachfolgenden Tabelle 1 genannten Pyrimidinderivate der allgemeinen Formel (I)

0184752

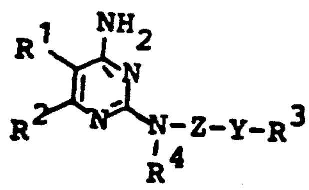

(I)

hergestellt werden.

Tabelle 1

| Beispiel Nr. | $R^1$ | $R^2$ | $R^3$ | $R^4$ | Z | Y | Phys. Konstante |
|---|---|---|---|---|---|---|---|
| 5 | Cl | $CH_3$ | $C_2H_5$ | H | $-(CH_2)_3-$ | 0 | Fp. 84°C |
| 6 | Cl | $CH_3$ | $C_3H_7$(i) | H | $-(CH_2)_3-$ | 0 | Öl |
| 7 | Cl | $CH_3$ | $CH_3$ | H | $-CH(CH_3)-(CH_2)_2-$ | 0 | Fp. 68-70°C |
| 8 | Cl | Cl | $C_2H_5$ | H | $-(CH_2)_3-$ | 0 | Isomerengemisch Fp. 67-70°C |
| 9 | Cl | Cl | $C_3H_7$(i) | H | $-(CH_2)_3-$ | 0 | Isomerengemisch Fp. 44-52°C |
| 10 | Cl | $CH_3$ | $CH_3$ | H | $-(CH_2)_3-$ | 0 | Fp. 66-68°C |
| 11 | Cl | Cl | $C_2H_5$ | H | $-CH(CH_3)-(CH_2)_2-$ | 0 | |
| 12 | Cl | Cl | $C_3H_7$(i) | H | $-CH(CH_3)-(CH_2)_2-$ | 0 | |
| 13 | Cl | Cl | $CH_3$ | H | $-CH(CH_3)-CH_2-$ | 0 | |
| 14 | Cl | Cl | $C_2H_5$ | H | $-CH(CH_3)-CH_2-$ | 0 | |

Tabelle 1 (Fortsetzung)

| Beispiel Nr. | $R^1$ | $R^2$ | $R^3$ | $R^4$ | Z | Y | Phys. Konstante |
|---|---|---|---|---|---|---|---|
| 15 | Cl | Cl | $C_3H_7$ | H | $-CH(CH_3)-CH_2-$ | O | |
| 16 | Cl | Cl | $CH_3$ | $CH_3$ | $-(CH_2)_2-$ | O | |
| 17 | Cl | Cl | $CH_3$ | $C_2H_5$ | $-(CH_2)_2-$ | O | |
| 18 | F | F | $CH_3$ | H | $-CH(CH_3)-(CH_2)_2-$ | O | |
| 19 | F | $OC_2H_5$ | $CH_3$ | H | $-CH(CH_3)-(CH_2)_2-$ | O | |
| 20 | Cl | Cl | $CH_3$ | H | $-CH(CH_3)-(CH_2)_2-$ | S | |
| 21 | F | $CF_3$ | $CH_3$ | H | $-(CH_2)_3-$ | O | Fp: 98°C |
| 22 | F | $CF_3$ | $CH_3$ | H | $-CH(CH_3)-(CH_2)_2-$ | O | Fp.: 95-97°C |
| 23 | F | $CF_3$ | $C_2H_5$ | H | $-(CH_2)_3-$ | O | |
| 24 | Cl | $CHCl_2$ | $CH_3$ | H | $-(CH_2)_3-$ | O | Fp.: 112-114°C |
| 25 | Cl | $CHCl_2$ | $C_2H_5$ | H | $-(CH_2)_3-$ | O | |
| 26 | Cl | $CHCl_2$ | $C_3H_7$(i) | H | $-(CH_2)_3-$ | O | Fp.: 80°C |

Tabelle 1 (Fortsetzung)

| Beispiel Nr. | $R^1$ | $R^2$ | $R^3$ | $R^4$ | Z | Y | Phys. Konstante |
|---|---|---|---|---|---|---|---|
| 27 | Cl | $CHCl_2$ | $CH_3$ | H | $-CH(CH_3)-(CH_2)_2-$ | O | |
| 28 | Cl | $CF_3$ | $CH_3$ | H | $-(CH_2)_3-$ | O | |
| 29 | Cl | $CF_3$ | $C_2H_5$ | H | $-(CH_2)_3-$ | O | |
| 30 | Cl | $CF_3$ | $C_3H_7$(i) | H | $-(CH_2)_3-$ | O | |
| 31 | Cl | $CF_3$ | $CH_3$ | H | $-CH(CH_3)-(CH_2)_2-$ | O | |
| 32 | Cl | Cl | $CH_3$ | H | $-CH_2-CH(C_3H_7(i))-CH_2-$ | O | $n_D^{20}$: 1.5478 |
| 33 | Cl | Cl | $CH_3$ | H | $-CH_2-CH(CH_3)-CH_2-$ | O | Fp.: 86°C |
| 34 | Cl | Cl | $CH_3$ | H | $-(CH_2)_3-$ | S | Fp.: 88°C |
| 35 | Cl | Cl | $-(CH_2)_4CH_3$ | H | $-(CH_2)_3-$ | O | Fp.: 37°C |
| 36 | Cl | Cl | $-(CH_2)_5CH_3$ | H | $-(CH_2)_3-$ | O | Fp.: 45°C |
| 37 | Cl | Cl | $CH_3$ | H | $-CH_2)_3-CH(CH_3)-$ | O | $n_D^{20}$: 1.5680 |

Tabelle 1 (Fortsetzung)

| Beispiel Nr. | $R^1$ | $R^2$ | $R^3$ | $R^4$ | Z | Y | Phys.Konstante |
|---|---|---|---|---|---|---|---|
| 38 | Cl | Cl | $-CH_2CH(CH_3)_2$ | H | $-(CH_2)_3-$ | O | Fp.: 72°C |
| 39 | Cl | Cl | $-C_4H_9(t)$ | H | $-(CH_2)_3-$ | O | Fp.: 80°C |
| 40 | Cl | Cl | $CH_3$ | $CH_3$ | $-(CH_2)_2-$ | S | Fp.: 108°C |
| 41 | Cl | Cl | $CH_3$ | H | $-C(CH_3)_2-CH_2-$ | O | |
| 42 | Cl | $-CH_2Cl$ | $CH_3$ | H | $-CH(CH_3)-(CH_2)_2-$ | O | Fp.: 110°C |

Ausgangsstoffe

Die als Ausgangsstoffe verwendeten Halogenpyrimidine der Formeln (II-1) bis (II-8) und deren Herstellung sind bekannt oder können in analoger Weise nach dort beschriebenen Verfahren erhalten werden:

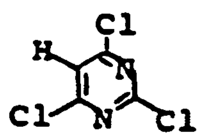 (II-1) Gabriel; Ber. 33, 3666 (1900).

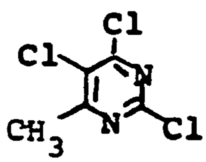 (II-2) R. Behrend; Justus Liebigs Ann. Chem. 229, 1 (1885).

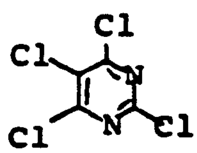 (II-3) S. C. Childress et al.; J. Am. Chem. Soc. 72, 4271 (1950).

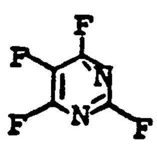 (II-4) E. Klauke et al.; J. Fluorine Chem. 21, 495 (1982).

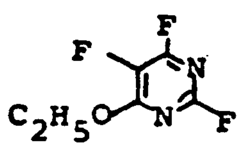 (II-5) H. Schröder et al.; J. org. Chem. 27, 2580 (1962).

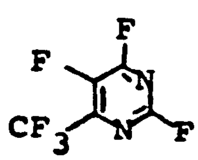 (II-6) E. Klauke et al.; J. Fluorine Chem. 21, 495 (1982).

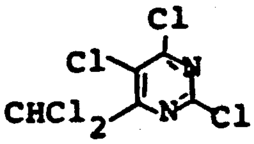
(II-7) DE-OS 3 131 735

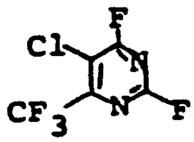
(II-8) E. Klauke et al.; J. Fluorine Chem. <u>21</u>, 495 (1982).

Die als Ausgangsstoffe verwendeten Aminopyrimidine der Formeln (VI-1) bis (VI-5) und deren Herstellung sind bekannt oder können in analoger Weise nach dort beschriebenen Verfahren erhalten werden:

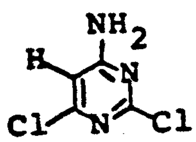
(VI-1) H. Bretschneider et al.; Monatsh. Chem. <u>92</u>, 128 (1961).

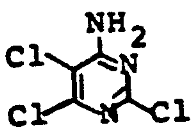
(VI-2) S. C. Childress et al.; J. Am. Chem. Soc. <u>72</u>, 4271 (1950).

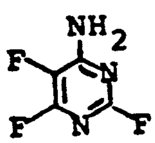
(VI-3) H. Schroeder et al.; J. Org. Chem. <u>27</u>, 2580 (1962).

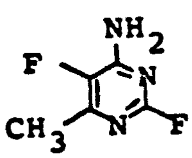
(VI-4) E. Klauke et al.; J. Fluorine Chem. <u>21</u>, 495 (1982).

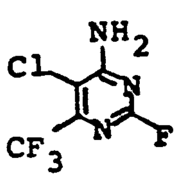
(VI-5) E. Klauke et al.; J. Fluorine Chem. <u>21</u>, 495 (1982).

0184752

Anwendungsbeispiele

In den folgenden Anwendungsbeispielen wurde die nachstehend aufgeführte Verbindung als Vergleichssubstanz herangezogen:

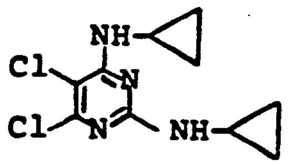

(VS-A)

(bekannt aus DE-OS 2 630 140, Beispiel 12).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen bei diesem Test z.B. die Verbindungen gemäß folgender Herstellungsbeispiele: (3), (4).

Patentansprüche

1. 2,4-Diaminopyrimidine der allgemeinen Formel (I)

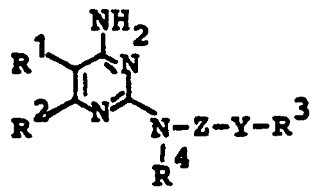

(I)

in welcher

$R^1$ für Wasserstoff oder Halogen steht,

$R^2$ für gegebenenfalls durch Halogen substituiertes Alkyl, Halogen oder Alkoxy steht,

$R^3$ für Alkyl steht,

$R^4$ für Wasserstoff oder Alkyl steht,

Y für Sauerstoff oder Schwefel steht und

Z für verzweigtes oder unverzweigtes Alkylen steht.

2. Pyrimidinderivate der allgemeinen Formel (I) gemäß Anspruch 1, dadurch gekennzeichnet, daß darin

$R^1$ für Wasserstoff, Chlor oder Fluor steht,

$R^2$ für Chlor, Fluor, Alkoxy mit 1-6 C-Atomen oder für gegebenenfalls durch Chlor und/oder Fluor substituiertes Alkyl mit 1-6 C-Atomen steht,

$R^3$ für Alkyl mit 1-6 C-Atomen steht,

$R^4$ für Wasserstoff oder Alkyl mit 1-6 C-Atomen steht,

Y für Sauerstoff oder Schwefel steht und

Z für eine verzweigte oder unverzweigte Alkylengruppe mit 2 bis 10 C-Atomen steht.

3. 4-Amino-2-(3-methoxypropylamino)-5,6-dichlorpyrimidin der Formel

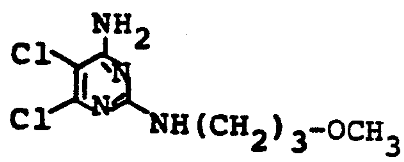

(3)

gemäß Anspruch 1.

4. 4-Amino-2-(3-methoxy-1-methylpropylamino)-5,6-dichlorpyrimidin der Formel

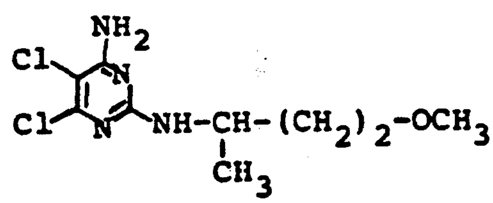

(4)

gemäß Anspruch 1.

5. Verfahren zur Herstellung von 2,4-Diaminopyrimidinen der allgemeinen Formel (I) gemäß Anspruch 1, dadurch gekennzeichnet, daß man

(A) Halogenpyrimidine der allgemeinen Formel (II)

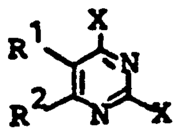

(II)

in welcher

$R^1$ und $R^2$ die oben angegebene Bedeutung haben, und X für Halogen (bevorzugt Fluor oder Chlor) steht,

zunächst mit einem Amin der Formel (III)

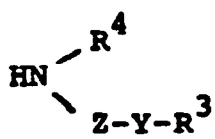

(III)

worin

$R^3$, $R^4$, Y und Z die oben angegebene Bedeutung haben,

in Gegenwart eines säurebindenden Mittels und gegebenenfalls in Gegenwart eines Verdünnungsmittels zu einem Gemisch der isomeren Pyrimidinderivate der allgemeinen Formel (IVa) und (IVb)

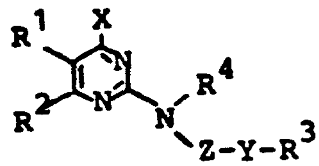 + 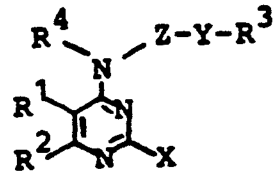

(IVa) (IVb)

worin

$R^1$, $R^2$, $R^3$, $R^4$, X, Y und Z die oben angegebene Bedeutung haben,

umsetzt (1. Stufe) und anschließend nach Trennung der beiden Isomeren das Pyrimidinderivat der Formel (IVa) in einem weiteren Reaktionsschritt mit Ammoniak (V)

$$NH_3 \qquad (V)$$

in Gegenwart eines säurebindenden Mittels und gegebenenfalls in Gegenwart eines Verdünnungsmittels zu Pyrimidinderivaten der Formel (I) umsetzt (2. Stufe),

oder

(B) 4-Aminopyrimidinderivate der allgemeinen Formel (VI)

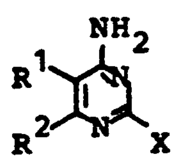

(VI)

worin $R^1$, $R^2$ und X die oben angegebene Bedeutung haben,

mit einem Amin der Formel (III) - wiederum unter den gleichen Reaktionsbedingungen wie bei Verfahren (A) - zum Pyrimidinderivat der Formel (I)

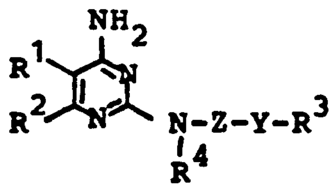

(I)

worin

$R^1$, $R^2$, $R^3$, $R^4$, Y und Z die oben angegebene Bedeutung haben,

umsetzt und für den Fall, wenn $R^2$ für Halogen steht, aus dem erhaltenen Gemisch der isomeren Pyrimidinderivate der allgemeinen Formel (I) und (Ia)

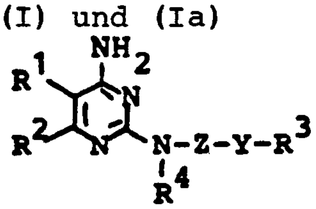

und

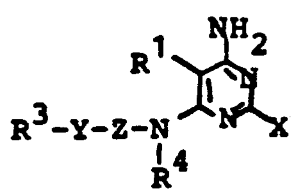

(I) (Ia)

worin $R^1$, $R^2$, $R^3$,$R^4$, X, Y und Z die oben angegebene Bedeutung haben,

das gewünschte Pyrimidinderivat der allgemeinen Formel (I) abtrennt.

6. Herbizide Mittel, gekennzeichnet durch einen Gehalt an mindestens einem 2,4-Diaminopyrimidin der allgemeinen Formel (I) gemäß Anspruch 1.

7. Verwendung von 2,4-Diaminopyrimidinen der allgemeinen Formel (I) gemäß Anspruch 1 zur Bekämpfung von unerwünschtem Pflanzenwuchs.

8. Verfahren zur Herstellung von herbiziden Mitteln, dadurch gekennzeichnet, daß man 2,4-Diaminopyrimidine der allgemeinen Formel (I) gemäß Anspruch 1 mit Streckmitteln und/oder oberflächenaktiven Mitteln vermischt.